(19) Europäisches Patentamt
European Patent Office
Office européen des brevets

(11) Numéro de publication : 0 305 302 B1

# (12) FASCICULE DE BREVET EUROPEEN

(45) Date de publication du fascicule du brevet : **18.09.91 Bulletin 91/38**

(51) Int. Cl.[5] : **C07C 69/593,** C07C 67/347

(21) Numéro de dépôt : **88420273.0**

(22) Date de dépôt : **29.07.88**

## (54) Perfectionnement au procédé de dimérisation catalytique d'un acrylate d'alkyle.

(30) Priorité : **19.08.87 FR 8711831**

(43) Date de publication de la demande : **01.03.89 Bulletin 89/09**

(45) Mention de la délivrance du brevet : **18.09.91 Bulletin 91/38**

(84) Etats contractants désignés : **AT BE CH DE ES FR GB IT LI LU NL SE**

(56) Documents cités :
**EP-A- 0 140 210**
**EP-A- 0 243 281**
**FR-A- 2 079 319**
**FR-A- 2 524 341**
**US-A- 4 638 084**

(73) Titulaire : **RHONE-POULENC CHIMIE**
**25, qual Paul Doumer**
**F-92408 Courbevoie Cédex (FR)**

(72) Inventeur : **Perron, Robert**
**La Pecollère**
**F-69390 Charly (FR)**
Inventeur : **Mutez, Sylvain**
**33, rue de Combemore 5, Hameau des Presles**
**F-69540 Irigny (FR)**

(74) Mandataire : **Varnière-Grange, Monique et al**
**RHONE-POULENC INTERSERVICES Service Brevets Chimie Centre de Recherches des Carrières B.P. 62**
**F-69192 Saint-Fons Cédex (FR)**

EP 0 305 302 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention se rapporte à un perfectionnement au procédé de dimérisation catalytique d'esters alkyliques de l'acide acrylique pour produire des diesters alkyliques de l'acide dihydromuconique.

Dans la demande de brevet européen n° 87420078.5 publié le 28/10/87 avec une date de priorité du 27/3/86 il a été proposé notamment un procédé de dimérisation catalytique d'un acrylate d'alkyle inférieur pour produire des diesters alkyliques de l'acide dihydromuconique par réaction à une température comprise entre 50 et 250°C d'un acrylate d'alkyle inférieur en présence d'un système catalytique comprenant du palladium ou un composé du palladium, caractérisé en ce que le système catalytique est formé à partir d'au moins :

a) — une source de palladium non halogénée,

b) — un composé du phosphore (III) de formule générale (I) :

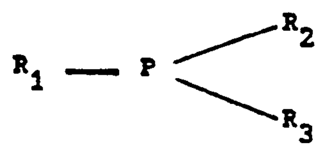

(I)

dans laquelle :

$R_1$, $R_2$ et $R_3$ représentent indépendamment un radical alkyle, cycloalkyle, aryle, alcoxy, cycloalcoxy ou aryloxy, l'un des radicaux $R_1$, $R_2$ ou $R_3$ pouvant, en outre, représenter un radical monovalent de formule générale (II) :

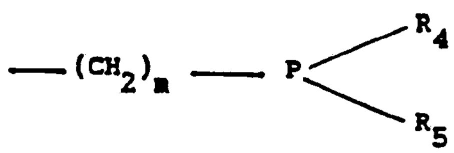

(II)

dans laquelle :

— m est un entier compris entre 1 et 4 inclus,

— $R_4$ et $R_5$ représentent indépendamment un radical alkyle, cycloalkyle, aryle, alcoxy ou aryloxy, et :

c) — d'au moins un hydracide HY dont l'anion associé $Y^-$ présente un caractère non coordinant vis-à-vis des ions palladium.

Les diesters ainsi produits sont des intermédiaires utiles, notamment, à la fabrication d'adipates d'alkyle voire d'acide adipique.

La matière de départ est un acrylate d'alkyle inférieur, c'est à dire dont le reste alkyle comporte de 1 à 8 atomes de carbone ; la dimension du groupe alkyle n'est pas critique et on recourt plus particulièrement à l'acrylate de méthyle ou à l'acrylate d'éthyle en raison de leur plus grande disponibilité. Le groupement alkyle peut comporter des substituants n'interférant pas avec la réaction recherchée.

Bien entendu, on peut utiliser comme matière de départ des produits du commerce non nécessairement purs.

Le système catalytique selon cette demande antérieure est formé à partir d'au moins une source de palladium non halogénée. Cette source de palladium, précurseur de l'entité catalytiquement active, peut être choisie parmi les diverses formes de palladium (O) ou de palladium (II) non halogénées. Parmi les formes de palladium (II) susceptibles de convenir à la mise en oeuvre dudit procédé, on peut citer les sels d'acides organiques tels l'acétate de palladium (II), le formiate de palladium (II), l'octanoate de palladium (II), l'éthylhexanoate de palladium (II) ; les sels d'acides minéraux tel le nitrate de palladium (II), des complexes $\pi$-allyliques de palladium (II) tel le diacétate de ($\pi$-allyl) palladium ; l'acétylacétonate de palladium (II).

On a préconisé l'emploi de l'acétate de palladium (II) ou de l'acétylacétonate de palladium (II), notamment en raison de leur plus grande disponibilité.

Parmi les sources de palladium (O) susceptibles de convenir à la mise en oeuvre dudit procédé, on peut citer le noir de palladium, le palladium déposé sur un support tel le charbon actif ou le gel de silice, les complexes du palladium et d'une trialkyl- ou triarylphosphine tel le tétrakis (triphénylphosphine) palladium.

On a déjà mis en évidence l'intérêt des composés du palladium (O) et de la dibenzylidèneacétone qui répondent à la formule générale (III) ci-après :

$$Pd_x(dba)_y \quad (III)$$

dans laquelle :
x est égal à 1 ou 2
dba représente un coordinat dibenzylidèneacétone,
y est égal à 2 ou à 3, y étant nécessairement égal à 3 lorsque x vaut 2.

Ces composés sont d'un accès facile. Ils peuvent être obtenus aisément par la réduction du chlorure de palladium en présence de dibenzylidèneacétone (dba) selon l'un quelconque des modes opératoires décerits par Y. ISHII et coll. dans Chem. Comm., 1970, p. 1065.

Les complexes $Pd(dba)_2$, $Pd_2(dba)_3$ et $Pd(dba)_3$ et leurs mélanges peuvent être utilisés indifféremment.

Dans cette même demande antérieure, il a également été indiqué qu'une concentration du palladium dans le milieu réactionnel d'au moins 0,1 millimole par mole d'acrylate paraît nécessaire pour obtenir un taux de transformation suffisant et qu'on n'observe pas d'avantage à dépasser la quantité de 3 millimoles par mole d'acrylate.

S'agissant du composé du phosphore (III), il a été précisé dans cette demande antérieure que sa quantité est en général telle que le rapport molaire P/Pd soit compris entre 1 et 15, un rapport molaire compris entre 1 et 3 ayant été préconisé.

S'agissant de l'hydracide HY dont l'anion associé $Y^-$ présente un caractère non coordinant vis-à-vis des ions palladium, préférence a été donnée à l'acide tétrafluoroborique et il a été indiqué qu'une quantité d'acide telle que le rapport $H^+/Pd$ soit compris entre 1 et 30 et, de préférence, entre 1 et 10 était requise, de bons résultats pouvant être obtenus pour un rapport $H^+/P$ de l'ordre de 2 à 5 environ.

La préparation ex-temporanée du système catalytique et sa préparation in-situ dans les conditions de la dimérisation ont été décrites, la première pouvant comporter la synthèse préalable d'un sel d'hydrogénophosphonium tel le tétrafluoroborate d'hydrogénotributylphosphonium.

Toutefois si l'intérêt de principe d'un tel procédé n'est pas mis en doute, son développement à l'échelle industrielle se heurte à au moins un des problèmes suivants :

— activité insuffisante du système catalytique,
— manque de sélectivité du système catalytique, qui se traduit en particulier, par l'apparition d'une proportion notable de produits lourds,
— manque de stabilité du palladium qui est susceptible de se déposer sur les parois du réacteur et qui est susceptible d'induire des variations importantes d'activité au cours du temps.

Ces divers problèmes deviennent plus particulièrement préoccupants lorsqu'on souhaite mettre en oeuvre le procédé dans des conditions de température et de concentration en palladium compatibles avec les exigences industrielles, c'est à dire une température comprise entre 70 et 150°C et une concentration en palladium comprise entre 0,1 et 3 mmol de palladium par mole d'acrylate d'alkyle.

Il est donc nécessaire de proposer un nouveau procédé de dimérisation des acrylates d'alkyle permettant d'obvier aux inconvénients précités.

La présente invention a donc pour objet un procédé de dimérisation des acrylates d'alkyle inférieur comprenant la mise en réaction d'au moins un acrylate d'alkyle inférieur en présence de palladium, d'acide tétrafluoroborique et d'un tétrafluoroborate d'hydrogénophosphonium, la concentration du palladium étant comprise entre 0,1 et 3 mmol par mole d'acrylate d'alkyle caractérisé en ce que la température de réaction est comprise entre 70 et 150°C et en ce que la concentration en tétrafluoroborate d'hydrogénophosphonium est supérieure ou égale à 6 mmol/par mole d'acrylate d'alkyle.

Par acrylate d'alkyle inférieur, on entend des composés au sens indiqué en tête du présent mémoire ; l'acrylate de méthyle convient plus particulièrement à la mise en oeuvre du présent procédé en raison de sa plus grande disponibilité.

Le palladium peut être introduit sous l'une quelconque des formes indiquées en tête du présent mémoire dans la mesure où ces composés ne renferment pas d'atome d'halogène.

Par tétrafluoroborate d'hydrogénophosphonium, on entend le sel issu de la réaction entre l'acide tétrafluoroborique et un composé du phosphore (III) répondant à la formule générale (I) en tête du présent mémoire.

Ce sel peut être représenté par la formule générale (IV) :

$$(HPR_1R_2R_3)^+ BF_4^- \quad (IV)$$

dans laquelle $R_1$, $R_2$ et $R_3$ ont les significations précédemment données.

Plus spécifiquement $R_1$, $R_2$ et $R_3$ identiques ou différents peuvent représenter :

— un radical alkyle contenant au maximum 8 atomes de carbone tels que les radicaux méthyle, éthyle, propyle, isopropyle, butyle, t-butyle et n-octyle,

— un radical cylcoalkyle contenant de 5 à 7 atomes de carbone tel qu'un radical cyclohexyle ou cycloheptyle,
— un radical aryle contenant de 6 à 12 atomes de carbone tels les radicaux phényle, p-toluyle, biphénylyle, naphtyle,
— un radical alcoxy comportant au plus 8 atomes de carbone tels les radicaux méthoxy et éthoxy,
— un radical aryloxy comportant de 6 à 12 atomes de carbone tel le radical phénoxy.

Conviennent plus particulièrement à la mise en oeuvre du procédé selon l'invention, le tétrafluoroborate d'hydrogénotributylphosphonium, le tétrafluoroborate d'hydrogénotricyclohexylphosphonium et le tétrafluoroborate d'hydrogénodiméthylphénylphosphonium.

La synthèse des sels en cause peut être réalisée extemporanément par addition, en milieu solvant, le cas échéant, de l'acide tétrafluoroborique (disponible sous forme d'une solution aqueuse ou sous forme de complexes avec des éthers tels l'éther éthylique et l'éther méthylique) sur le composé du phosphore (III) correspondant. Le solvant de cette synthèse peut être le substrat mis en oeuvre dans la réaction de dimérisation. Bien entendu, cette synthèse peut être également réalisée en présence de palladium ou de ses composés.

La concentration en palladium est comprise entre 0,1 et 3 mmol par mole d'acrylate d'alkyle. Pour une bonne mise en oeuvre du procédé selon l'invention, elle sera avantageusement comprise entre 0,2 et 1,5 mmol par mole d'acrylate d'alkyle.

La température de la réaction de dimérisation est comprise entre 70 et 150°C ; pour une bonne mise en oeuvre du procédé, elle sera comprise entre 90 et 130°C.

La concentration en tétrafluoroborate d'hydrogénophosphonium est supérieure à 6 mmol par mole d'acrylate d'alkyle. On n'observe pas d'avantage particulier à dépasser une concentration de l'ordre de 200 mmol par mole d'acrylate d'alkyle. Cette concentration sera avantageusement comprise entre 10 et 100.

Toutefois pour une bonne mise en oeuvre du procédé selon l'invention, le seuil minimum de cette concentration sera d'autant plus élevé que la température de réaction choisie sera élevée.

Le procédé selon l'invention est également conduit en présence d'acide fluoroborique. La quantité d'acide à mettre en oeuvre sera généralement comprise 0,5 et 50 mmol par mole d'acrylate d'alkyle et, de préférence, comprise entre 2 et 15 mmol par mole d'acrylate d'alkyle.

La durée de réaction (ou temps de séjour) est généralement comprise entre 10 minutes et 8 heures.

Les produits de réaction peuvent alors être récupérés par distillation après neutralisation du milieu réactionnel et, le cas échéant, une première distillation pour assurer l'élimination du tiers solvant lorsque cela est nécessaire.

Les exemples ci-après illustrent l'invention.

Dans les exemples les conventions suivantes sont utilisées :

| | |
|---|---|
| Bu = | n-butyle |
| AM = | Acrylate de méthyle |
| $Pd(acac)_2$ = | acétylacétonate de palladium |
| $[HBF_4]$ = | concentration de l'acide tétrafluoroborique en excès |
| t = | durée de l'essai à la température de travail et au moment de la prise d'échantillon |
| RR = | nombre de moles du produit considéré pour 100 moles d'acrylate de méthyle engagées |
| ME-2G = | méthylène-2 glutarate de méthyle |
| DHM = | $\Delta^2$— et $\Delta^3$— dihydromuconate de méthyle |
| [Pd] soluble = | concentration du palladium soluble en mmol par kilogramme de mélange réactionnel au bout du temps t, la concentration initiale en palladium soluble étant indiquée dans la colonne intitulée t = 0. |
| (*) = | polymérisation massive du milieu réactionnel. |

Exemples 1 à 8 — Essai témoin (a)

Dans un réacteur en acier inoxydable NSMC d'une capacité de 50 $cm^3$, purgé à l'argon, on introduit successivement le précurseur palladié en solution dans l'acrylate de méthyle distillé, le tétrafluoroborate d'hydrogénotributylphosphonium et l'excès d'acide tétrafluoroborique complexé à l'éther éthylique (les concentrations des différents constituants du système catalytique sont exprimées en mmoles par mole d'acrylate de méthyle chargé).

Après fermeture du réacteur et mise sous agitation, la température du milieu réactionnel est portée à 110°C.

L'évolution de la réaction est suivie par la prise d'échantillons au cours du temps que l'on analyse par chromatographie en phase gazeuse pour déterminer la nature et les proportions des produits formés et par absorption atomique pour doser le palladium soluble.

Les conditions particulières ainsi que les résultats obtenus sont rassemblés dans le tableau ci-après annexé.

Exemple 9 :

Dans le réacteur décrit ci-avant et selon un mode opératoire analogue au précédent, on charge :

— de l'acrylate de méthyle,
— de l'acétylacétonate de palladium à raison de 0,3 mmol par mole d'acrylate de méthyle,
— du tétrafluoroborate d'hydrogénotributylphosphonium à raison de 31,5 mmol par mole d'acrylate de méthyle,
— de l'acide tétrafluoroborique complexé à l'éther éthylique à raison de 12,6 mmol par mole d'acrylate de méthyle.

La température de réaction est de 130°C. Les résultats obtenus sont indiqués ci-après en usant des mêmes conventions que celles précédemment définies :

| t | RR % | | [Pd] soluble mmol/kg | |
|---|---|---|---|---|
| | ME-2G | DHM | t = 0 | t |
| 50 mn | 0,2 | 6,2 | 7,4 | 7,4 |
| 380 mn | 1,8 | 72 | 7,4 | 7,2 |

Exemple 10 :

Le mode opératoire décrit pour les exemples 1 à 8 ci-avant a été modifié par le fait qu'on a introduit successivement :

— de l'acrylate de méthyle (279 mmol),
— de l'acétylacétonate de palladium à raison de 0,68 mmol par mole d'acrylate de méthyle et
— de l'acide tétrafluoroborique complexé à l'éther éthylique à raison de 19,7 mmol par mole d'acrylate de méthyle et qu'on a refroidi puis rajouté goutte à goutte de la triphénylphosphine : à raison de 17 mmol par mole d'acrylate de méthyle.

Après cette introduction, on a porté le milieu réactionnel à 110°C et procédé aux mêmes déterminations que dans le cadre des exemples 1 à 8.

Les résultats obtenus sont indiqués ci-après en usant des mêmes conventions que celles précédemment définies :

| t | RR % |  | [Pd] soluble mmol/kg |  |
| --- | --- | --- | --- | --- |
|  | ME-2G | DHM | t = 0 | t |
| 85 mn | 0,6 | 23,8 | 3,2 | 3,3 |
| 385 mn | 1,7 | 53,5 | " | 3 |

| Ex. n° | [$HPBu_3BF_4$] mmol/mole AM | $Pd(acac)_2$ mmol/mole AM | [$HBF_4$] mmol/mole AM | t | RR % ME-2G | RR % DHM | [Pd] soluble mmol/kg t = 0 | [Pd] soluble mmol/kg t |
|---|---|---|---|---|---|---|---|---|
| 1 | 6 | 0,6 | 2,5 | 55mn | 0,4 | 21 | 6,4 | 6,3 |
| | | | | 4h 25mn | 1,7 | 77 | | 6,4 |
| 2 | | 1,2 | 2,5 | 55mn | 1,3 | 56 | 13,6 | 13,7 |
| | | | | 3h 55mn | 2 | 85 | | 13,4 |
| 3 | | 0,3 | 2,4 | 55mn | 0,3 | 6 | 3,8 | 3,7 |
| | | | | 6h 15mn | 0,5 | 24 | | 3,6 |
| a | 30 | 0,6 | 0 | | (*) | (*) | | |
| 4 | 32,7 | 0,65 | 2 | 55mn | - | 2 | 6,8 | 6,6 |
| | | | | 7h 20mn | 0,6 | 22,5 | | 6,1 |
| 5 | 30 | 0,6 | 3,6 | 55mn | 0,5 | 24,5 | 6,4 | 5,6 |
| | | | | 7h 25mn | 1,4 | 60,5 | | 5,6 |
| 6 | 30,5 | 0,4 | 6 | 55mn | 0,3 | 19 | 6,4 | 3,9 |
| | | | | 6h 25mn | 1 | 41 | | 4,3 |
| 7 | 42,6 | 0,57 | 2,3 | 55mn | 0,2 | 9 | 5,8 | 5,8 |
| | | | | 7h 25mn | 1,2 | 40,5 | | 5,9 |
| 8 | 62 | 0,62 | 2,5 | 60mn | 0,1 | 3,2 | 5,9 | 6,0 |
| | | | | 4h 25mn | 0,7 | 16 | | 5,9 |

## Revendications

1. Procédé de dimérisation des acrylates d'alkyle inférieur comprenant la mise en réaction d'au moins un acrylate d'alkyle inférieur en présence de palladium, d'acide tétrafluoroborique et d'un tétrafluoroborate d'hydrogénophosphonium, la concentration du palladium étant comprise entre 0,1 et 3 mmol par mole d'acrylate d'alkyle caractérisé en ce que la température de réaction est comprise entre 70 et 150°C et en ce que la concentration en tétrafluoroborate d'hydrogénophosphonium est supérieure ou égale à 6 mmol par mole d'acrylate d'alkyle.

2. Procédé selon la revendication 1, caractérisé en ce que le tétrafluoroborate d'hydrogénophosphonium répond à la formule (IV)

$$(HPR_1R_2R_3)^+ BF_4^- \quad (IV)$$

dans laquelle :

$R_1$,$R_2$ et $R_3$ représentent indépendamment un radical alkyle, cycoalkyle, aryle, alcoxy, cycloalcoxy ou aryloxy, l'un des radicaux $R_1$, $R_2$ ou $R_3$ pouvant, en outre, représenter un radical monovalent de formule générale (II) :

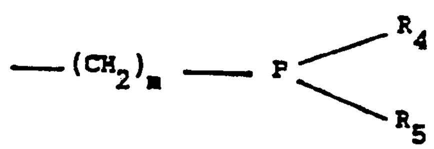

(II)

dans laquelle :

— m est un entier compris entre 1 et 4 inclus,

— $R_4$ et $R_5$ représentent indépendamment un radical alkyle, cycloalkyle, aryle, alcoxy ou aryloxy,

3. Procédé selon la revendication 2, caractérisé en ce que dans la formule (IV), $R_1$, $R_2$ et $R_3$ identiques ou différents représentent :

— un radical alkyle contenant au maximum 8 atomes de carbone,

— un radical cycloalkyle contenant de 5 à 7 atomes de carbone,

— un radical aryle contenant de 6 à 12 atomes de carbone,

— un radical alcoxy comportant au plus 8 atomes de carbone ou

— un radical aryloxy comportant de 6 à 12 atomes de carbone.

4. Procédé selon l'une quelconque des revendications précédentes caractérisé en ce que la concentration en palladium est comprise entre 0,2 et 1,5 mmol par mole d'acrylate d'alkyle.

5. Procédé selon l'une quelconque des revendications précédentes caractérisé en ce que la température de la réaction est comprise entre 90 et 130°C.

6. Procédé selon l'une quelconque des revendications précédentes caractérisé en ce que la concentration en tétrafluoroborate d'hydrogénophosphonium est comprise entre 10 et 100 mmol par mole d'acrylate d'alkyle.

7. Procédé selon l'une quelconque des revendications précédentes caractérisé en ce que la quantité d'acide fluoroborique est comprise entre 0,5 et 50 mmol par mole d'acrylate d'alkyle.

8. Procédé selon la revendication 7, caractérisé en ce que la quantité d'acide fluoroborique est comprise entre 2 et 15 mmol par mole d'acrylate d'alkyle.

9. Procédé selon l'une quelconque des revendications précédentes caractérisé en ce que le tétrafluoroborate d'hydrogénophosphonium est le tétrafluoroborate d'hydrogénotributylphosphonium.

10. Procédé selon l'une quelconque des revendications précédentes caractérisé en ce que l'acrylate d'alkyle inférieur est l'acrylate de méthyle.

## Claims

1. Process for the dimerisation of lower alkyl acrylates comprising the reaction of at least one lower alkyl acrylate in the presence of palladium, tetrafluoroboric acid and a hydrogenophosphonium tetrafluoroborate, the palladium concentration being between 0.1 and 3 mmol per mole of alkyl acrylate, characterised in that the reaction temperature is between 70 and 150°C and in that the hydrogenophosphonium tetrafluoroborate concentration is greater than or equal to 6 mmol per mole of alkyl acrylate.

2. Process according to Claim 1, characterised in that the hydrogenophosphonium tetrafluoroborate corresponds to the formula (IV)

$$(HPR_1R_2R_3)^+ BF_4^- \quad (IV)$$

in which :

$R_1$, $R_2$ and $R_3$ denote, independently, an alkyl, cycloalkyl, aryl, alkoxy, cycloalkoxy or aryloxy radical, it being possible for one of the radicals $R_1$, $R_2$ or $R_3$, in addition, to denote a monovalent radical of the general formula (II) :

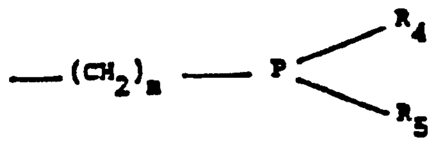

(II)

in which :

m is an integer between 1 and 4 inclusive,

$R_4$ and $R_5$ denote, independently, an alkyl, cycloalkyl, aryl, alkoxy or aryloxy radical,

3. Process according to Claim 2, characterised in that, in the formula (IV), $R_1$, $R_2$ and $R_3$, which may be identical or different, denote :

an alkyl radical containing at most 8 carbon atoms,

a cycloalkyl radical containing from 5 to 7 carbon atoms,

an aryl radical containing from 6 to 12 carbon atoms,

an alkoxy radical containing at most 8 carbon atoms, or

an aryloxy radical containing from 6 to 12 carbon atoms.

4. Process according to any one of the preceding claims, characterised in that the palladium concentration is between 0.2 and 1.5 mmol per mole of alkyl acrylate.

5. Process according to any one of the preceding claims, characterised in that the reaction temperature is between 90 and 130°C.

6. Process according to any one of the preceding claims, characterised in that the hydrogenophosphonium tetrafluoroborate concentration is between 10 and 100 mmol per mole of alkyl acrylate.

7. Process according to any one of the preceding claims, characterised in that the quantity of fluoroboric acid is between 0.5 and 50 mmol of alkyl acrylate.

8. Process according to Claim 7, characterised in that the quantity of fluoroboric acid is between 2 and 15 mmol per mol of alkyl acrylate.

9. Process according to any one of the preceding claims, characterised in that the hydrogenophosphonium tetrafluoroborate is tributylphosphonium tetrafluoroborate.

10. Process according to any one of the preceding claims, characterised in that the lower alkyl acrylate is methyl acrylate.

**Patentansprüche**

1. Verfahren zur Dimerisierung von niederen Alkylacrylaten, umfassend die Umsetzung von mindestens einem niederen Alkylacrylat in Gegenwart von Palladium, Tetrafluorborsäure und einem Hydrogenphosphonium-tetrafluorborat, wobei die Konzentration an Palladium zwischen 0,1 und 3 mmol pro mol Alkylacrylat liegt, dadurch gekennzeichnet, daß die Reaktionstemperatur zwischen 70 und 150°C liegt und daß die Konzentration an Hydrogenphosphonium-tetrafluorborat größer oder gleich 6 mmol pro mol Alkylacrylat ist.

2. Verfahren nach Anspruch 1, dadurch gekennzeichnet, daß das Hydrogenphosphonium-tetrafluorborat der Formel (IV) entspricht

$$(HPR_1R_2R_3)^+ BF_4^- \quad (IV)$$

in der :

$R_1$, $R_2$ und $R_3$ unabhängig voneinander einen Alkyl-, Cycloalkyl-, Aryl-, Alkoxy-, Cycloalkoxy- oder Aryloxyrest bedeuten, einer der Reste $R_1$, $R_2$ oder $R_3$ unter anderem einen einwertigen Rest der allgemeinen Formel

(II)

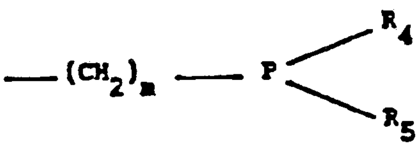

(II)

bedeuten kann, in der

— m eine ganze Zahl von 1 bis 4 bedeutet,

— $R_4$ und $R_5$ unabhängig voneinander einen Alkyl-, Cycloalkyl-, Aryl-, Alkoxy- oder Aryloxyrest bedeuten.

3. Verfahren nach Anspruch 2, dadurch gekennzeichnet, daß in der Formel (IV) $R_1$, $R_2$ und $R_3$ gleich oder verschieden sind und bedeuten :

— einen Alkylrest mit bis zu 8 Kohlenstoffatomen,

— einen Cycloalkylrest mit 5 bis 7 Kohlenstoffatomen,

— einen Arylrest mit 6 bis 12 Kohlenstoffatomen,

— einen Alkoxyrest mit bis zu 8 Kohlenstoffatomen oder

— einen Aryloxyrest mit 6 bis 12 Kohlenstoffatomen.

4. Verfahren nach einem der vorangehenden Ansprüche, dadurch gekennzeichnet, daß die Konzentration an Palladium zwischen 0,2 und 1,5 mmol pro mol Alkylacrylat beträgt.

5. Verfahren nach einem der vorangehenden Ansprüche, dadurch gekennzeichnet, daß die Reaktionstemperatur zwischen 90 und 130°C liegt.

6. Verfahren nach einem der vorangehenden Ansprüche, dadurch gekennzeichnet, daß die Konzentration ein Hydrogenphosphonium-tetrafluorborat zwischen 10 und 100 mmol pro mol Alkylacrylat liegt.

7. Verfahren nach einem der vorangehenden Ansprüche, dadurch gekennzeichnet, daß die Menge an Fluorborsäure zwischen 0,5 und 50 mmol pro mol Alkylacrylat beträgt.

8. Verfahren nach Anspruch 7, dadurch gekennzeichnet, daß die Menge an Fluorborsäure zwischen 2 und 15 mmol pro mol Alkylacrylat beträgt.

9. Verfahren nach einem der vorangehenden Ansprüche, dadurch gekennzeichnet, daß das Hydrogenphosphonium-tetrafluorborat Hydronegotributylphosphonium-tetrafluorborat ist.

10. Verfahren nach einem der vorangehenden Ansprüche, dadurch gekennzeichnet, daß das niedere Alkylacrylat Methylacrylat ist.